# EUROPÄISCHE PATENTANMELDUNG

(11) **EP 1 462 437 A1**
(43) Veröffentlichungstag der Anmeldung: **29.09.2004**
(21) Anmeldenummer: 04006185.5
(22) Anmeldetag: 16.03.2004
(51) Int. Cl.: C07C 67/56, C07C 69/28, C07C 69/30, C07C 29/76, C07C 41/36, C07C 45/79, C07C 51/47

(54) **Verfahren zur Aufreinigung von Verbindungen mit funktionellen Gruppen**

(30) Priorität: 28.03.2003 DE 10314078
(71) Anmelder: Nutrinova Nutrition Specialties & Food Ingredients GmbH, 65929 Frankfurt am Main (DE)
(72) Erfinder: Fabritius, Dirk, Dr., 55116 Mainz (DE); Neumann, Doreen, 65719 Hofheim (DE)
(74) Vertreter: Schweitzer, Klaus, Dr.

(57) **Zusammenfassung**

Die vorliegende Erfindung betrifft ein Verfahren zur Gewinnung einer mindestens eine funktionelle Gruppe tragenden, gesättigten, organischen Verbindungen aus einem Gemisch, welches diese gesättigte organische Verbindung mit mindestens einer funktionellen Gruppe sowie eine oder mehrere andere organische Verbindungen enthält, wobei man:
i) einen mit Silberionen beladenen Ionenaustauscher mit dem Gemisch bei einer Temperatur, die unterhalb des Siedepunktes der Mischung liegt, vermischt
ii) anschließend den Überstand entfernt und
iii) die an den Ionenaustauscher angelagerte gesättigte organische Verbindung mit mindestens einer funktionellen Gruppe vom Ionenaustauscher ablöst.

## Beschreibung

Die Erfindung betrifft ein Verfahren zur Gewinnung einer mindestens eine funktionelle Gruppe tragenden, gesättigten, organischen Verbindungen aus einem Gemisch, welches diese gesättigte organische Verbindung mit mindestens einer funktionellen Gruppe sowie eine oder mehrere andere organische Verbindungen enthält, wobei man
i) einen mit Silberionen beladenen Ionenaustauscher mit dem Gemisch bei einer Temperatur, die unterhalb des Siedepunktes der Mischung liegt, vermischt
ii) anschließend den Überstand entfernt und
iii) die an den Ionenaustauscher angelagerte, gesättigte, organische Verbindung mit mindestens einer funktionellen Gruppe mit einem Lösungsmittel vom Kationentauscher ablöst.

Die Trennung und Aufreinigung von organischen Verbindungen aus Substanzgemischen ist nach wie vor ein in vielen Bereichen nicht zufriedenstellend gelöstes Problem. Die Abtrennung von bestimmten Verunreinigungen oder die Trennung von Mischungen komplexer Stoffgemische bedürfen spezieller Verfahren. Der Erfolg der Trennung hängt wesentlich vom Aufbau der zu trennenden Verbindung ab; insbesondere für Verbindungen mit ähnlicher Struktur benötigt man besonders angepaßte, oftmals sehr kostspielig Verfahren. So stehen z.B. für Partialester des Glycerins mit höheren Fettsäuren wie Monoglyceride kaum einfache und wirksame Verfahren für eine Aufreinigung zur Verfügung.

Monoglycerinester z.B. der Ölsäure, Palmitinsäure, Stearinsäure stehen im Blickpunkt wirtschaftlichen Interesses. Sie besitzen emulgierende, stabilisierende, plastifizierende und verdickende Eigenschaften und werden beispielsweise als Emulgatoren für Teige, für Süß- und Backmittel, Margarine und Speiseeis in der Nahrungsmittelindustrie eingesetzt (Kennzeichnung für Mono- und Diglyceride von Fettsäuren: E 471). Ebenso finden sie beispielsweise als Gleitmittel in der Kunststoffverarbeitung (z.B.: Glycerinmonostearat) Anwendung.

Durch Umsetzung von Monoglyceriden mit Essigsäure erhält man acetylierte, flüssige bis wachsartige Monoglyceride (Acetoglyceride), die aufgrund ihrer guten Verträglichkeit mit pharmazeutischen Wirkstoffen und ihrer physiologischen Unbedenklichkeit in Lebensmitteln als Schutzfilme, in Kosmetika und Pharmaprodukten eingesetzt werden.

Besonders pharmazeutische Anwendungen treten in letzter Zeit verstärkt in das öffentliche Interesse. So werden Monoglyceride als PPAR-Aktivatoren (PPAR: Peroxisome Proliferating Factor) beschrieben, die geeignet sind, Übergewichtigkeit und Diabetes zu kurieren (JP-A 2001-354558).

Monoglyceride werden in der Regel durch Umesterung von Triglyceriden mit Glycerin oder durch Umsetzung von Glycerin mit entsprechenden Fettsäuren erhalten. Aber auch die heute weitverbreitete Lipase-Reaktion (KR-A 127510, JP-A 09-268299) wird zur Darstellung von Monoglyceriden und Diglyceriden eingesetzt. Meist werden Mischungen aus Triglyceriden, Diglyceriden, Monoglyceriden, freien Fettsäuren und Glycerin erhalten, und in fast allen Fällen ist ein Aufreinigen, das heißt eine Trennung und Anreicherung der Monoglyceride aus den komplexen Gemischen notwendig.

Häufig werden dazu Destillationsverfahren verwendet (Bornscheuer et al., Enzymes in Lipid Modification Ed. U. T. Bomscheuer, Wiley-VCH, 2000), die aber durch die bei diesen Verfahren benötigten hohen Temperaturen (T > 200 °C) nur eingeschränkt nutzbar sind, da Acylmigrationen oder sogar die Zerstörung der gewünschten Produkte, insbesondere bei oxidationsempfindlichen Verbindungen die Folge sind (Naohiro et al. US-A 6,025,348, Mares et al. CS-A 86-4520). Dies erfordert ein aufwendiges Downstreaming für die Gewinnung reiner Monoglyceride (Bomscheuer et al. s.o.).

Ein weiteres gängiges Verfahren zur Aufreinigung ist die Isolierung über Kälte-Kristallisation, da Monoglyceride in der Regel andere Schmelzpunkte aufweisen als die korrespondierenden Triglyceride, Diglyceride und freien Fettsäuren. In einigen Fällen gelingt dabei die selektive Abtrennung der Monoglyceride von Diglyceriden, Triglyceriden und anderen Bestandteilen.

Diese Methode ist jedoch nicht universell einsetzbar, da uneinheitliche Gemische schwierig aufzuarbeiten sind, z.B. Monoglyceride mit unterschiedlichen Fettsäuren oder Monoglyceride, die als Regioisomere vorliegen. Auch die Prochiralität vieler Monoglyceride spielt eine wesentliche Rolle. So unterscheiden sich die Schmelzpunkte von rac-1-Palmitoylglycerin (D,Lalpha Glycerinmonopalmitat, ca. +74,9 °C) und 3-Palmitoyl-sn-glycerin (L-alpha-Glycerinmonopalmitat, +71,1 °C). Der Schmelzpunkt von 2-Monopalmitat (ca. +68 °C) ist ebenfalls unterschiedlich. Der Umstand, daß der Schmelzpunkt wesentlich durch den Fettsäurerest beeinflußt wird, erschwert die selektive Kristallisation, insbesondere aus Gemischen.

In der Praxis gelingt lediglich die Abtrennung über die Kristallisation von Monoglyceriden, die Fettsäuren mit mehr als 12 Kohlenstoffatomen tragen. Darüber hinaus wird die Trennung bei Monoglyceriden mit ungesättigten Fettsäuren wie z.B. Ölsäure (18: 1) erheblich schwieriger. Bei mehrfach ungesättigten Fettsäuren gelingt die Trennung (Kristallisation) praktisch nicht mehr.

Kang et al (KR-A 94-6988) beschreiben die synthetische Herstellung von Monoglyceriden und die anschließende Aufreinigung mittels Lösungsmittelextraktion.

Kolstad et al beschreiben eine Methode zur Reinigung von Monoglyceriden mittels Flüssigflüssig-Extaktionsverfahren (US-A 5,959,128).

Die Aufreinigung durch Säulenchromatographie wird von Mekawa et al (JP-A 61-166399) beschrieben. Ihm gelingt in geringen Ausbeuten die Auftrennung von alpha- und beta-Monoglyceriden bei Verwendung des Ionenaustauschmaterials XAD, einem Ionenaustauscher auf Kunstharzbasis. Im allgemeinen ist die Verwendung von Kieselgelen als Ionenaustauschermaterial nur wenig geeignet, da durch das saure Verhalten vieler Kieselgele eine Acylmigration katalysiert werden kann und somit einer Aufreinigung entgegenwirkt.

Die PCT-Anmeldung 02/06158 beschreibt ein Extraktionsverfahren durch selektive Komplexierung an einem mit Silberionen beladenen Kationentauscher und anschließender Dekomplexierung für die Gewinnung von ungesättigten gegebenenfalls. derivatisierten Verbindungen aus Gemischen. Dieses Verfahren ist auf Verbindungen mit hochungesättigten Komponenten, wie z.B. ungesättigte langkettige Fettsäuren beschränkt.

Keines der bekannten Verfahren führt zu zufriedenstellenden Ausbeuten und hinreichenden Selektivitäten bei der Aufreinigung von Gemischen von Verbindungen der Eingangs beschriebenen Art.

Es bestand somit die Aufgabe, ein Verfahren zur Verfügung zu stellen, welches die Gewinnung von gesättigten organischen Verbindungen mit funktionellen Gruppen aus Gemischen, welche diese gesättigten, organischen Verbindungen mit funktionellen Gruppen sowie eine oder mehrere andere organische Verbindungen enthalten, gestattet. Dieses Verfahren sollte eine quantitativ ausreichende Aufreinigung ermöglichen, eine Vereinfachung des Verfahrens und eine ökonomische Ausgestaltung desselben erlauben. Außerdem sollte das Verfahren selektiv sein, um beispielsweise die Trennung von sich strukturell nur wenig unterscheidenden Verbindungen zu ermöglichen.

Diese Aufgabe wird gelöst durch ein Verfahren zur Gewinnung einer mindestens eine funktionelle Gruppe tragenden, gesättigten, organischen Verbindungen aus einem Gemisch, welches diese gesättigte organische Verbindung mit mindestens einer funktionellen Gruppe sowie eine oder mehrere andere organische Verbindungen enthält, wobei man:
i) einen mit Silberionen beladenen Ionenaustauscher mit dem Gemisch bei einer Temperatur, die unterhalb des Siedepunktes der Mischung liegt, vermischt
ii) anschließend den Überstand entfernt und
iii) die an den Ionenaustauscher angelagerte, gesättigte, organische Verbindung mit mindestens einer funktionellen Gruppe mit einem Lösungsmittel vom Kationentauscher ablöst.

Unter einer "mindestens eine funktionelle Gruppe tragenden, gesättigten, organischen Verbindung" wird erfindungsgemäß eine gesättigte, organische Verbindung verstanden, die mindestens eine Gruppe enthält, die ausgewählt ist aus =O, -OH, -C(O)OH, -C(O)H, -COOR, -C-O-C- und -C-O-R- (R= organischer Rest). Bevorzugt sind solche Verbindungen, die aktive Wasserstoffatome tragen, wie Carbonsäuren, Hydroxycarbonsäuren, Ketocarbonsäuren, Alkohole, Carbonsäurester, Ether und Ketone. Beispiele für solche Verbindungen sind langkettige, verzweigte und nicht verzweigte, gesättigte Alkohole mit 12 bis 30 C-Atomen, wie Lauryl-, Myristyl- Stearylalkohol und deren Derivate, wie Ester und Ether. Ebenfalls bevorzugt sind cyclische, gesättigte Alkohole, wie z.B. Stigmastanol, Ergostanol, Cholestanol sowie Stannole mit weiteren OH-Gruppen und/oder unterschiedlichen Seitenketten (z.B. ethylverzweigt, methylverzweigt etc.).

Ebenso bevorzugt sind Ester von Hydroxycarbonsäuren z.B. der Glycolsäure, Milchsäure, Weinsäure oder Hydroxybuttersäure, aber auch Aminocarbonsäuren mit langkettigen, verzweigten oder nicht verzweigten, gesättigten Alkoholen mit 12 bis 30 C-Atomen.

Weitere bevorzugte Verbindungen sind Derivate (z.B. Ester, Ether, Addukte) mehrwertiger Alkohole wie z.B. Ethylenglycol, Propylenglycol, Propandiol, 1,2- oder 1,3-Butandiol; Glycerin-Ether mit langkettigen, gesättigten Alkoholen, bevorzugt Fettalkoholen oder Glycerin-Ester mit langkettigen, gesättigten Carbonsäuren, bevorzugt Fettsäuren. Besonders bevorzugt sind Mono- und Diester des Glycerins mit gesättigten Fettsäuren wie z.B. Laurin-, Myristin-, Palmitin-, Palmitolein-, Stearin-, Arachin- und Behensäure. Weiterhin bevorzugt sind Monound Dialkylether des Glycerins wie z.B. Chimyl- (=Monoether d. Glycerins mit einem C16-Alkohol) und Batylalkohol (=Monoether d. Glycerins mit einem C18-Alkohol).

Die erfindungsgemäß zu trennenden Verbindungen kommen gewöhnlich in Mischungen mit einer oder mehren organischen Verbindungen vor. Diese organischen Verbindungen können z.B. Strukturisomere der anzureichernden Verbindungen, Gemische verschiedener Substanzklassen (z.B. Ether und Ester) oder Gemische homologer Reihen (z.B. Ester die unterschiedliche Fettsäuren, z.B. C18 oder C16 gebunden haben), Stellungsisomere oder Regioisomere sein.

Unter einem "mit Silberionen beladenen Ionenaustauscher" versteht man einen porösen Träger, auf dessen Oberfläche sich Silberionen befinden. Zweckmäßigerweise sollte die Beladung mit Ag⁺-Ionen bei 100% der theoretisch möglichen Beladung liegen. Da die maximale Silberbeladung vom jeweiligen Tauscher abhängt, läßt sich keine allgemein gültige Kapazität angeben. Die Beladung mit einem Kation wird üblicherweise in meq/g (meq=Milliäquivalent) oder meq/ml wet (Milliäquivalente/Milliliter Feuchtgewicht) angegeben. Als Beispiel beträgt die maximale Silberionenbeladung eines Amberlite 15 Kationenaustauschers 4,6 meq/g bzw. 1,8 meq/ml.

Bevorzugt handelt es bei den porösen Trägem um Kationenaustauscher. Erfindungsgemäß einsetzbare Kationenaustauscher sind insbesondere solche, die stark saure Eigenschaften haben. Gele, die als Grundkörper Styrol mit Divinylbenzolverzweigungen aufweisen, als aktive Silbertragende Gruppe Sulfonsäure- und/oder Carboxylgruppen tragen und microporös oder bevorzugt macroporös sind, sind besonders bevorzugt. Insbesondere sind auch macroreticulare Ionenaustauscher geeignet, da sie lösungsmittelstabil sind und im Vergleich zu Gelen eine wesentlich größere Oberfläche aufweisen. Diese tragen ebenfalls Sulfonsäure- und/oder Carboxylgruppen. Beispiele für solche Ionenaustauscher sind: Dowex® 50 WX8, Dowex® 50 WX4, Dowex® 50 WX2, Dowex® MWC1, Dowex® MSC1, Dowex® Monosphere C-350, Dowex® CCR-2, Dowex® DR 2030, Amberlite® CG50Amberlite® IR 120, Amberlyst® 15, Bio-Rex® 70 Resin, Macherey & Nagel Ps-DVB®. Besonders bevorzugt sind Amberlyst® 15 und Dowex® DR 2030.

Die Herstellung der silberbeladenen Ionentauscher erfolgt zweckmäßigerweise in Anlehnung an das von Nieto et al. (Nieto, S., A. M. Cordoba; J. Sanhuenzy and A. Valenzuela (1997): Obtention of highly purified fractions of eicosapentaenoic acid and docosahexaenoic acid from sardine oil by silver-resin chromatography: A semi-preparative prodcedure. Grasas y Aceites, 48(4), 197-199) für Dowex® 50WX8 (frühere Bezeichnung Dowex® W-HCR-W2) beschriebene Verfahren. Das Verfahren wird jedoch vereinfacht bzw. abgewandelt. Es erfolgt weder eine Vorbereitung des Trägermaterials in einer beheizbaren Glassäule, noch wird eine Vorwaschung des Trägermaterials mit organischem Lösungsmittel durchgeführt. In dem erfindungsgemäßen Verfahren ist im Gegensatz zu dem von Nieto et al. offenbarten Verfahren die Korngröße des Trägermaterials ausschlaggebend für die Qualität der Trennung und die Ausbeute. Es zeigt sich eine Abhängigkeit von der Menge der angelagerten Verbindung und der Korngröße des verwendeten Materials. So wird bei Korngrößen größer 50 mesh keine Anlagerung von Monoglyceriden an einen mit Silberionen vollständig beladenen (= 100% Beladung) Kationentauscher mehr gefunden. Größere Partikel liefern eher schlechtere Ergebnisse (bis hin zu gar keiner Anlagerung, wahrscheinlich wegen zu geringer Silberbeladung), kleinere dagegen nicht viel bessere, dafür aber Verfahrensprobleme. Bevorzugt werden Korngrößen von 20 bis 50 mesh, bevorzugt 100-400 mesh, ganz besonders bevorzugt 200-400 mesh, die eine ausreichende Komplexierung gewährleisten.

Dabei reicht die Beladungskapazität der erfindungsgemäß einsetzbaren Ionenaustauscher von 0,1 bis 15 Gew.-% .

Die Selektivität läßt sich in Abhängigkeit von der zu trennenden Verbindung steuern. Im allgemeinen gilt folgendes. Je mehr freie p-Elektronen (Valenzen) eine Verbindung aufweist, umso stärker bindet sie an den versilberten Tauscher (Komplexbindung Ag⁺ mit den p-Elektronen). Dabei ist auch die Verfügbarkeit der Elektronen wichtig, d.h. je zugänglicher (sterisch nicht behindert) die Elektronen sind, um so leichter die Komplexbindung. Aromatische Verbindungen lassen sich nicht so leicht komplexieren wie lineare Olefine. Mit der Auswahl des Lösungsmittels läßt sich ebenfalls die Trennung steuern. Je polarer das Lösungsmittel desto stärker muß die Komplexbindung ausgeprägt sein, damit eine Verbindung nicht vom Tauscher abgelöst wird. Die Stärke wird zum einen von der Zugänglichkeit der p-Elektronen beeinflußt und zum anderen von der Anzahl der Komplexbindungen. Je mehr Komplexbindungen, desto stärker die Wechselwirkung. So lassen sich z.B. Monoglyceride von Diglyceriden abtrennen, da Monoglyceride eine bessere Komplexbindung der freien p-Elektronen der Sauerstoffe aus der Alkoholgruppe zulassen. Je unpolarer das Lösungsmittel, umso schwächer kann die Wechselwirkung sein, damit eine Verbindung noch mit dem Tauscher wechselwirkt. Um die Verbindungen abzulösen reicht in der Regel die Verwendung eines polareren Lösungsmittels aus, als das was bei der Anlagerung verwendet wurde. Gegebenenfalls wird noch bei erhöhter Temperatur in demselben Lösungsmittel gearbeitet. Sollte die Wechselwirkung zu stark sein, kann auf jeden Fall mit Acetonitril abgelöst werden. Ähnliches gilt für die Temperatur. Je höher die Temperatur, umso stärker muß die Wechselwirkung sein, damit eine Verbindung am Tauscher verbleibt. Generell sollte bei Raumtemperatur (ca. 26°C) gearbeitet werden. Die Ablösung gelingt dann bei Temperaturen unterhalb des Siedepunktes des Lösungsmittels.

Die Eigenschaften des Ionenaustauschers sind ebenfalls wichtig. So zeigen macroreticulare Ionenaustauscher die besten Trennergebnisse bei hydrophoben Verbindungen. Dies liegt besonders an der Porosität und den Oberflächeneigenschaften, die mehr Austauschergruppen auf der Oberfläche aufweisen als es bei Geltypen der Fall ist. Je hydrophober ein Tauscher, umso besser die Anlagerung von lipophilen Verbindungen.

Die Zeit ist ebenfalls eine Variante die zur Selektivitätssteigerung eingesetzt werden kann. Generell gilt, je kürzer die Anlagerungszeit, desto höher die Reinheiten der primär an den Tauscher angelagerten Verbindung.

Ebenfalls einen großen Einfluß auf die Aktivität des Kationentauschers hat der Gehalt an Wasser im Kationentauscher. Für eine Erhaltung einer hohen Aktivität des Kationentauschers ist es zweckmäßig, den Wassergehalt des Kationentauschers möglichst niedrig, bevorzugt unter 10 ppm, besonders bevorzugt unter 5 ppm, insbesondere unter 3 ppm zu halten.

Bei dem erfindungsgemäßen Verfahren geht man zweckmäßigerweise wie folgt vor: Zunächst wird das zu trennende Gemisch in einem organischen Lösungsmittel gelöst. Geeignete Lösungsmittel zur Anlagerung der Verbindung an den Ionenaustauscher sind beispielsweise Alkohole, Ketone, Ether, Ester, Diketone, Diester, Diether, Diole, Polyole, Nitrile und Dinitrile, bevorzugt lebensmittelrechtlich zugelassene Lösungsmittel wie Hexan, Ethanol, Aceton oder Isopropanol oder ein Gemisch aus zwei oder mehreren dieser Lösungsmittel. Besonders bevorzugt sind Methylisobutylketon und Ethanol.

Das gelöste Substanzgemisch wird dann zu dem Ionenaustauscher gegeben. Zweckmäßigerweise stellt man eine Suspension aus gelöstem Substanzgemisch und Ionenaustauscher her. Die Suspension wird dann bei Raumtemperatur (ca. 26°C) 0,5 bis 5,0 Stunden, bevorzugt 1,0 bis 3,5 Stunden, insbesondere 1,2 bis 1,7 Stunden mit dem Ionenaustauscher kontaktiert. Es hat sich als vorteilhaft erwiesen, die Suspension während dieser Zeit zu rühren oder zu schütteln. Zur Unterstützung des Komplexierungsprozesses kann es von Vorteil sein, der Suspension Wärme zuzuführen, um eine höhere Reinheit der komplexierten Verbindung zu erreichen, d.h. die Selektivität des Verfahrens zu erhöhen. Eine Erwärmung auf > 40 °C bis unterhalb des Siedepunktes des verwendeten Lösemittel ist vorteilhaft. Bevorzugt ist ein Temperaturbereich von 40 bis 80 °C.

Es kann weiterhin von Vorteil sein, den Komplexierungsprozess unter Schutzgasatmosphäre, z.B. Argon, durchzuführen, um unerwünschte Reaktionen z.B. mit Luftsauerstoff oder Luftfeuchtigkeit zu verhindern, die zur Deaktivierung des Ionenaustauschers und damit zu ungenügender Komplexierung der gewünschten Substanz führen können.

Die an den Ionenaustauscher angelagerte Verbindung kann nun nach Abdekantieren des Überstandes von dem Ionenaustauscher getrennt und somit isoliert werden. Zweckmäßigerweise geschieht dies mit Hilfe eines geeigneten Lösungsmittels. Als Lösungsmittel zur Ablösung der Verbindung vom Ionenaustauscher können Alkohole, bevorzugt Ethanol, Ether, Ketone, Ester, Nitrile oder ein Gemisch derartiger Lösungsmittel eingesetzt werden. In der Regel wird bei der Anlagerung kälteres Lösungsmittel verwendet als bei der Ablösung. Zweckmäßigerweise wird bei der Anlagerung ein unpolareres Lösungsmittel verwendet als bei der Ablösung. Ein ideales Lösungsmittel für die Ablösung ist Acetonitril, dies hat jedoch den Nachteil, daß es toxisch ist. Besonders bevorzugt ist Ethanol (Warm für die Ablösung und kalt für die Anlagerung). Für die Anlagerung eignet sich ebenfalls bevorzugt Hexan.

Alternativ kann auch die ,nicht gewünschte' Verbindung selektiv an den Ionenaustauscher gebunden werden, wobei sich dann die gewünschte Verbindung im Überstand anreichert und dort isoliert werden kann. Welche Variante des erfindungsgemäßen Verfahrens bevorzugt ist, hängt von Art, Anzahl und Position der funktionellen Gruppe ab. In der Regel lagert sich die polarere Verbindung an den Tauscher an und die unpolarere verbleibt im Überstand. Aber auch die Zugänglichkeit an die funktionelle Gruppe ist wichtig, so lagern sich 1-Monoglyceride deutlich besser an den Tauscher an als 2-Monoglyceride, da dort die beiden verbliebenen OH-Gruppen abgeschirmt werden durch den Fettsäurerest. So lassen sich z.B. in einfacher Weise Triglyceride mit gesättigten Fettsäuren von Monoglyceriden mit einer gesättigten Fettsäure abtrennen, da die Triglyceride nicht an den Tauscher anlagern.

Mit Hilfe des erfindungsgemäßen Verfahrens lassen sich Verbindungen aus komplexen Stoffgemischen durch eine einfache Anlagerung an einen Ionenaustauscher in hohen Reinheiten und Ausbeuten aufkonzentrieren. Im Gegensatz zu Chromatographieverfahren, bei denen eine Trennung erst bei einer bestimmten Bodenzahl bzw. nach Einstellen einer unterUmständen sehr hohen Zahl an Gleichgewichten erreicht wird, stellt sich nach dem erfindungsgemäßen Verfahren nur ein einziges Gleichgewicht während des Komplexierungsprozesses ein. Technische Probleme, die normalerweise durch den Wechsel des Lösungsmittel in der Chromatographie auftreten (Luftblasen, Quellen des Tauschers, Inhomogenitäten etc.) treten bei dem erfindungsgemäßen Verfahren nicht auf. Ebenfalls treten keine Flussprobleme (inhomogener Fluß) über die Trennsäule (Gradientenbildung) auf. Hohe Reinheiten des Produktes können mit dem erfindungsgemäßen Verfahren einfach erreicht werden.

Auf einfache Art lassen sich besonders bevorzugt Gemische von Monoglyceriden und Diglyceriden ( z.B. 1:1 Mischung von 1,2- und 1,3-Dipalmitat) aufreinigen.

Besonders überraschend ist, daß sich das Verfahren ebenfalls für Gemische bestehend aus Monoglyceriden mit an unterschiedlichen Positionen substituierten Fettsäuren hervorragend eignet. So läßt sich beispielsweise ein Gemisch aus 1- Monomyristat und 2-Monopalmitat durch die selektive Bindung von 1-Monomyristat an den Kationentauscher in einfacher Weise abtrennen.

Das erfindungsgemäße Verfahren stellt eine enorme Vereinfachung und ökonomische Verbesserung der im Stand der Technik beschriebenen Aufreinigungsverfahren dar.

Im Folgenden wird die Erfindung anhand von Beispielen näher beschrieben. Dabei wurden folgende Meßmethoden verwendet:

### Gaschromatographische Analyse

Die Analyse der Öle erfolgt nach Umesterung durch allgemein bekannte Methoden (z.B. methanolische Salzsäure) in die Methylester und anschließender gaschromatographischer Analyse (Hewlett-Packard GC6890, Säule: Macherey & Nagel FFAP Permabond 0,1 µm (25 m, 0,25 mm), Splitbetrieb (10:1), Trägergas: Helium (constant flow 1,0 ml/min), FID-Betrieb mit Wasserstoff (30 ml/min) und Sauerstoff (300 ml/min) als Brenngase, Make up: 20 ml Helium, Detektor- und Injektortemperatur: jeweils 225° C, GC-Ofentemperaturprogramm: Anfangstemperatur 160° C, Haltephase 12 Minuten isotherm, Temperaturanstiegsrate 10° C/min auf Endtemperatur 230° C, diese für 5 min halten, Injektionsvolumen; 1,0 ml). Durch Zugabe eines internen Standards (z.B. eine gesättigter Fettsäuremethylester wie Heptadecansäuremethylester [C17](dieser lagert sich nicht an)) zum Reaktionsansatz kann eine quantitative Analyse durchgeführt werden; die Angabe erfolgt in Area-%.

### Herstellung von zu 100 % mit Silberionen beladenem Amberlyst® 15 (20-50 mesh)

20 g Amberlyst® 15 werden in eine Nutsche oder Glassäule mit Nutsche gegeben und mit 1 M Natriumnitrat-Lösung (NaNO₃) solange gespült, bis der pH-Wert des Eluates von sauer nach neutral umschlägt. Die Neutralisation zeigt die verminderte Bildung von Salpetersäure an, die beim Tausch von Protonen zu Natrium-Ionen gebildet wird. Ist der Kationentauscher vollständig mit Natrium-Ionen beladen, bleibt das Eluat neutral.

Jetzt kann auf zwei unterschiedliche Weise weiter verfahren werden. Entweder wird der mit Natriumionen beladene Kationentauscher solange mit 0,4 M Silbemitrat-Lösung gespült bis im Eluat Silberionen nachweisbar sind, oder der Kationentauscher wird zuerst in einem Rund- oder Erlenmeyerkolben mit Natriumnitrat-Lösung überspült. Die überschüssige Natriumnitrat-Lösung wird dann verworfen. Im nachfolgenden wird mit 5,4 ml 0,4 M Silbemitratlösung/g Amberlyst® 15 für 8-12 h gerührt. Der Überstand wird entfernt.
Der Kationentauscher (ca. 2,0 mmol Ag⁺/ml H⁺-Tauscher, wobei 1 g H⁺-Dowex ca. 0,9 ml H⁺ Dowex entspricht) wird dreimal mit 100 ml Wasser silberionenfrei gewaschen und anschließend zweimal mit 100 ml Ethanol wasserfrei gewaschen; dazu wird der Ansatz 1 Stunde lang gerührt. Nachfolgend läßt man in 100 ml Acetonitril über Nacht (12 h) stehen. Danach wird erneut zweimal mit jeweils 100 ml Ethanol gewaschen. Der Ionenaustauscher kann jetzt verwendet werden. Acetonitril kann auch durch dreimaliges Verwenden von 100 ml Ethanol ersetzt werden.

### Beispiel 1

### Auftrennung einer Mischung aus 1-Monomyristat und eines 1,2/1,3-Dipalmitatgemisches

Da die Analyse von Fettsäuren in der Regel einfacher und schneller verläuft, wurde in diesem Beispiel die Selektivität des Tauschers in Bezug auf die Anlagerung von Monoglyceriden und Diglyceriden mit verschiedenen Fettsäuren durchgeführt. So läßt sich relativ schnell erkennen, ob Monoglyceride (MG) oder Diglyceride (DG) bevorzugt angelagert werden.

Es werden 181,2 mg 1-Monomyristat (Sigma-Aldrich) und 173,1 mg Dipalmitatgemisch (1:1 Mischung 1,2-DG und 1,3-DG, Sigma-Aldrich) in 120 ml Methylisobutylketon gelöst und zu 52,1 g vollversilbertem Amberlyst@ 15 Ionenaustauscher gegeben. Die Analyse des Einsatzgemisches weist einen Myristinsäuregehalt von 47,0 Gew.-% und einen Palmitinsäuregehalt von 53,0 Gew.- % auf. Es wird 90 Minuten bei Raumtemperatur (26 °C) mit 100 U/min im Erlenmeyerkolben geschüttelt.

Anschließend wird der Überstand abgenommen und der Tauscher dreimal mit 50 ml Methylisobutylketon gewaschen. Die vereinigten Lösungsmittelextrakte werden am Rotationsverdampfer eingeengt. Man erhält 265,0 mg eines festen weißen Rückstandes. Die GC-Analyse zeigt einen Myristinsäuregehalt von 37,6 Gew.-% und einen Palmitinsäuregehalt von 62,4 Gew.-%.

Den Tauscher wäscht man dreimal mit jeweils 100 ml Ethanol für eine Stunde aus. Die Überstände werden vereinigt und ebenfalls eingeengt. Man isoliert 101,2 mg eines weißen festen Produktes. Die GC-Analyse nach Umesterung der beiden Fraktionen (Überstand Methylisobutylketon und Waschfraktion Ethanol) ergibt folgendes Bild:

Die Produktfraktion zeigt eine Reinheit an Myristinsäure von 76 Gew.-% und für Palmitinsäure von 24,4 Gew.-%. Es ist zu erkennen, daß es sich bei dem angelagerten Material um überwiegend 1-Monomyristat handelt, da fast ausschließlich Myristinsäure detektiert wurde.

**Tabelle 1**

| | 1-Monomyristat | 1,2- und 1,3-Dipalmitat (1:1 Mischung) | Summe |
|---|---|---|---|
| | 181,2 mg | 173,1 mg | |
| GC-Analyse(Area-%) | 47,0 % | 53,0 % | |
| Überstand | | | 265,0 mg |
| GC-Analyse(Area-%) | 37,6 % | 62,4 % | |
| Amberlyst * | | | 101,2 mg |
| GC-Analyse(Area-%) | 76,0% | 24,4 % | |

| | | | |
|---|---|---|---|
| * nach Losen vom Ionenaustauscher | | | |

### Beispiel 2: Auftrennung eines Gemisches von 1-Monostearat und 2-Monopalmitat

Es werden 50,0 mg 2-Monopalmitat (Sigma-Aldrich) und 51,0 mg 1-Monostearat (Sigma-Aldrich) in 100 ml Methylisobutylketon gelöst und zu 10 g vollversilbertem Amberlyst® 15 Ionenaustauscher gegeben. Die Analyse des Einsatzgemisches weist einen Stearinsäuregehalt von 48,0 Gew.-% und einen Palmitinsäuregehalt von 51,2 Gew.-% auf. Es wird 90 Minuten bei Raumtemperatur (26° C) mit 100 U/min im Erlenmeyerkolben geschüttelt.

Anschließend wird der Überstand abgenommen und der Tauscher fünfmal mit 100 ml Methylisobutylketon gewaschen. Die vereinigten Lösungsmittelextrakte werden am Rotationsverdampfer eingeengt. Man erhält 61,6 mg eines festen weißen Rückstandes. Die GC-Analyse zeigt einen Palmitinsäuregehalt von 47,2 Gew.-% und einen Stearinsäuregehalt von 46,6 Gew.- % .

Den Tauscher wäscht man dreimal mit jeweils 100 ml Ethanol für eine Stunde aus. Die Überstände werden vereinigt und ebenfalls eingeengt. Man isoliert 49,2 mg eines weißen, festen Produktes. Die GC-Analyse nach Umesterung der beiden Fraktionen (Überstand Methylisobutylketon und Waschfraktion Ethanol) ergibt folgendes Bild:

Die Produktfraktion zeigt eine Reinheit an Stearinsäure von 57,5 Gew.-% und für Palmitinsäure von 36,2 Gew.-%. Es ist zu erkennen, daß es sich bei dem angelagerten Material um angereichertes 1 -Monostearat handelt, da hauptsächlich Stearinsäure detektiert wurde.

**Tabelle 2**

| | 1-Monostearat | 2-Monopalmitat | Summe |
|---|---|---|---|
| | 51,0 mg | 50,0 mg | |
| GC-Analyse(Area-%) | 48,0 % | 51,2 % | |
| Überstand | | | 61,6 mg |
| GC-Analyse(Area-%) | 46,6 % | 47,2 % | |
| Amberlyst * | | | 49,2 mg |
| GC-Analyse(Area-%) | 57,5% | 36,7 % | |

| | | | |
|---|---|---|---|
| * nach Lösen vom Ionenaustauscher | | | |

Verändert man das eingesetzte Verhältnis 1-Monostearat zu 2-Monopalmitat von 5,1:4,8 auf 4,5:5,4 so lassen sich Produktfraktionen isolieren, die einen Stearinsäuregehalt von über 70% aufweisen.

## Patentansprüche

1. Verfahren zur Gewinnung einer mindestens eine funktionelle Gruppe tragenden, gesättigten, organischen Verbindungen aus einem Gemisch, welches diese gesättigte organische Verbindung mit mindestens einer funktionellen Gruppe sowie eine oder mehrere andere organische Verbindungen enthält, wobei man:
i) einen mit Silberionen beladenen Ionenaustauscher mit dem Gemisch bei einer Temperatur, die unterhalb des Siedepunktes der Mischung liegt, vermischt
ii) anschließend den Überstand entfernt und
iii) die an den Ionenaustauscher angelagerte gesättigte organische Verbindung mit mindestens einer funktionellen Gruppe vom Ionenaustauscher ablöst.

2. Verfahren nach Anspruch 1, **dadurch gekennzeichnet, daß** die eine funktionelle Gruppe tragende, gesättigte, organische Verbindung mindestens eine Gruppe enthält, die ausgewählt ist aus =O, -OH, -C(O)OH, -C(O)H, -COOR, -C-O-C- und -C-O-R- (R= organischer Rest).

3. Verfahren nach Anspruch 1 oder 2, **dadurch gekennzeichnet, daß** die eine funktionelle Gruppe tragende, gesättigte, organische Verbindung mindestens ein aktives Wasserstoffatom trägt.

4. Verfahren nach Anspruch 1, 2 oder 3, **dadurch gekennzeichnet, daß** die eine funktionelle Gruppe tragende, gesättigte, organische Verbindung ausgewählt wird aus Carbonsäuren, Hydroxycarbonsäuren, Ketocarbonsäuren, Alkoholen, Carbonsäurestern, Ethem und Ketonen.

5. Verfahren nach einem der Ansprüche 1 bis 4, **dadurch gekennzeichnet, daß** die eine funktionelle Gruppe tragende, gesättigte, organische Verbindung ausgewählt wird aus:
Alkoholen mit 12 bis 30 C-Atomen, Estern von Hydroxycarbonsäuren und/oder Aminocarbonsäuren und Estern oder Ethern von mehrwertigen Alkoholen.

6. Verfahren nach Anspruch 5, **dadurch gekennzeichnet, daß** die eine funktionelle Gruppe tragende, gesättigte, organische Verbindung ausgewählt wird aus: Estern oder Ethem von Ethylenglycol, Propylenglycol, Propandiol, 1,2- oder 1,3-Butandiol und Glycerin.

7. Verfahren nach Anspruch 6, **dadurch gekennzeichnet, daß** die eine funktionelle Gruppe tragende, gesättigte, organische Verbindung ausgewählt wird aus: Chimyl-, Batyl-Selachylalkohol.

8. Verfahren nach Anspruch 6, **dadurch gekennzeichnet, daß** die eine funktionelle Gruppe tragende, gesättigte, organische Verbindung ausgewählt wird aus: mono- und disubstituiertem Glycerin, wobei die Substituenten gleiche oder verschiedene Fettsäuren sind.

9. Verfahren nach einem der Ansprüche 1 bis 8, **dadurch gekennzeichnet, daß** der Ionenaustauscher ein Kationentauscher ist.

10. Verfahren nach einem der Ansprüche 1 bis 9, **dadurch gekennzeichnet, daß** der Ionenaustauscher saure Eigenschaften aufweist und microporös, macroporös oder macroreticular ist.

11. Verfahren nach Anspruch 10, **dadurch gekennzeichnet, daß** der Kationentauscher aus der folgenden Liste ausgewählt ist: Dowex® 50 WX8, Dowex® 50 WX4, Dowex(R) 50 WX2, Dowex® M WC 1, Dowex® MSC 1, Dowex® Monosphere C-350, Dowex® CCR-2, Dowex® DR 2030, Amberlite® CG50, Amberlite® IR 120, Amberlyst® 15, Bio-Rex® 70 Resin, Macherey&Nagel Ps-DVB®.

12. Verfahren nach Anspruch 11, **dadurch gekennzeichnet, daß** als Kationentauscher Amberlyst® 15 oder Dowex® DR 2030 eingesetzt wird.

13. Verfahren nach einem der Ansprüche 1 bis 12, **dadurch gekennzeichnet, daß** die an den Ionenaustauscher angelagerte gesättigte organische Verbindung mit mindestens einer funktionellen Gruppe vom Ionenaustauscher mit einem Lösungsmittel abgelöst wird.

14. Verfahren nach Anspruch 13, **dadurch gekennzeichnet, daß** das Lösungsmittel ausgewählt ist aus der Gruppe umfassend Alkohole, Ether, Ketone, Ester, Nitrile und Mischungen aus zwei oder mehreren dieser Lösungsmittel.

15. Verfahren nach Anspruch 14, **dadurch gekennzeichnet, daß** das Lösungsmittel Ethanol ist.

16. Verfahren nach einem der Ansprüche 1 bis 15, **dadurch gekennzeichnet, daß** das Gemisch in einem Lösungsmittel gelöst ist, welches ausgewählt ist aus einer oder mehreren Verbindungen der Gruppe bestehend aus: Alkanen, Ketonen, Ethern, Estern, Diketonen, Diestern, Diethern, Diolen, Polyolen, Nitrilen, Dinitrilen und Alkoholen.
